(19) **Europäisches Patentamt**
**European Patent Office**
**Office européen des brevets**

(11) **EP 4 245 845 A1**

(12) **EUROPEAN PATENT APPLICATION**
published in accordance with Art. 153(4) EPC

(43) Date of publication:
**20.09.2023 Bulletin 2023/38**

(21) Application number: **21891984.3**

(22) Date of filing: **12.11.2021**

(51) International Patent Classification (IPC):
***C12N 5/071*** *(2010.01)*

(52) Cooperative Patent Classification (CPC):
**C12N 5/06**

(86) International application number:
**PCT/JP2021/041694**

(87) International publication number:
**WO 2022/102739 (19.05.2022 Gazette 2022/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.11.2020 JP 2020188489**

(71) Applicant: **ADEKA CORPORATION
Arakawa-ku
Tokyo
116-8554 (JP)**

(72) Inventors:
• **HOMMA, Mitsumasa**
**Tokyo 116-8554 (JP)**

• **HIWATARI, Kenichiro**
**Tokyo 116-8554 (JP)**
• **OBARA, Haruki**
**Tokyo 116-8554 (JP)**
• **KIMURA, Takuya**
**Tokyo 116-8554 (JP)**
• **KINOSHITA, Keita**
**Tokyo 116-8554 (JP)**

(74) Representative: **Gille Hrabal
Partnerschaftsgesellschaft mbB
Patentanwälte
Brucknerstraße 20
40593 Düsseldorf (DE)**

Remarks:
The complete document including Reference
Table(s) and the Sequence Listing(s) can be
downloaded from the EPO website

(54) **DECELLULARIZED TISSUE COMPOSITION**

(57) The object of the present invention is to provide a decellularized tissue that has appropriate strength and exhibits good tissue regeneration.

The problem can be solved by a decellularized tissue composition comprising a decellularized tissue and a protein A with (a) molecular weight of 30,000 to 70,000 and (b) isoelectric point of pI 6.00 to 9.00

EP 4 245 845 A1

**Description**

TECHNICAL FIELD

The present invention relates to a decellularized tissue composition.

BACKGROUND ART

When transplanting a graft derived from a living tissue of another person or another species of animal, graft rejection by the recipient tissue is problematic. The development of artificial tissues is expected to be a solution to this problem. Various polymers have been tested as materials for artificial tissues, but the incompatibility of these materials with living tissues has led to problems such as dropout and infection at the joint site between the graft and the living tissue.

In order to improve the compatibility of grafts with biological tissues, techniques have been developed to use decellularized tissues consisting of the remaining supportive tissues (extracellular matrix, ECM) after removal of cells from biological tissues as grafts.

Decellularization means the removal of cellular components such as nucleic acids that are antigenic to the recipient, thereby avoiding immune rejection.

Decellularized tissues can be prepared, for example, by decellularizing living tissues using a treatment solution containing a surfactant (Patent literatures 1 to 3).

CITATION LIST

PATENT LITERATURE

[Patent literature 1] JP 2005-514971 A
[Patent literature 2] JP 2006-507851 A
[Patent literature 3] JP 2005-531355 A

SUMMARY OF INVENTION

TECHNICAL PROBLEM

When decellularized tissue is used in regenerative medicine, it is important that the tissue can be quickly regenerated in addition to the absence of rejection. Decellularized tissues having appropriate strength and good tissue regeneration are desired.

Therefore, the object of the present invention is to provide a decellularized tissue that has appropriate strength and exhibits good tissue regeneration.

SOLUTION TO PROBLEM

The present inventors have conducted intensive studies into a decellularized tissue that has appropriate strength and exhibits good tissue regeneration, as a result, surprisingly found that a decellularized tissue composition containing specific protein(s) have excellent strength and cell adhesiveness, and promote good tissue regeneration.

The present invention is based on the above findings.
Accordingly, the present invention relates to:

[1] a decellularized tissue composition comprising a decellularized tissue and a protein A with (a) molecular weight of 30,000 to 70,000 and (b) isoelectric point of pI 6.00 to 9.00,
[2] the decellularized tissue composition of item [1], further comprising a protein B with (a) molecular weight of 3,000 to 15,000 and (b) isoelectric point of pI 3.00 to 5.50,
[3] the decellularized tissue composition of item [1] or [2], wherein an amount of the protein A is 0.01 to 5.0% by weight with respect to the decellularized tissue composition,
[4] the decellularized tissue composition according to any one of items [1] to [3], wherein an amount of the protein B is 0.001 to 3.0% by weight with respect to the decellularized tissue composition,
[5] the decellularized tissue composition according to any one of items [1] to [4], wherein a mass ratio of protein A and protein B in the composition (A:B) is 5:1 to 150:1,

[6] the decellularized tissue composition according to any one of items [1] to [5], wherein a DNA amount in dry mass is 0.0300 % by weight or less with respect to the decellularized tissue composition,

[7] the decellularized tissue composition according to any one of items [1] to [6], wherein the protein A is annexin, and

[8] the decellularized tissue composition of any one of items [1] to [7], wherein the protein B is a peptide derived from fibromodulin.

ADVANTAGEOUS EFFECTS OF INVENTION

The decellularized tissue composition of the present invention can exhibit good tissue regeneration ability (e.g., cell attraction effect and cell differentiation induction effect) in vivo because of its appropriate strength and excellent cell adhesiveness. In addition, the superior strength of the composition provides excellent handling performance during surgery.

BRIEF DESCRIPTION OF THE DRAWINGS

[Fig. 1] Fig. 1 is photographs showing the spots of different proteins in Example 1 and Comparative Example 1 by two-dimensional electrophoresis of decellularized tissues in Example 1 and Comparative Example 1.
[Fig.2] Fig. 2 is graphs showing the results of cell adhesion tests of decellularized tissue compositions of Examples 1 to 13 and Comparative Examples 1 and 3.
[Fig. 3] Fig. 3 is a graph showing the modulus of elasticity at 10% elongation of decellularized tissue compositions of Examples 1 and 11 and Comparative Example 1.
[Fig. 4] Fig. 4 is a graph showing the maximum modulus of decellularized tissue compositions of Examples 1 and 11 and Comparative Example 1.
[Fig. 5] Fig. 5 is a graph showing the breaking strength of decellularized tissue compositions of Examples 1 and 11 and Comparative Example 1.
[Fig. 6] Fig. 6 is a graph showing the suture strength of decellularized tissue compositions of Examples 1 and 11 and Comparative Example 1.
[Fig. 7] Fig. 7 is photographs showing the spots of proteins in decellularized tissues of Examples 9 and 11, which are different from those of comparative Example 1, by two-dimensional electrophoresis.

DESCRIPTION OF EMBODIMENTS

The decellularized tissue composition of the present invention comprises a decellularized tissue and a protein with (a) molecular weight of 30,000 to 70,000 and (b) isoelectric point of pI 6.00 to 9.00 (hereinafter referred to as a protein A). The decellularized tissue composition of the present invention may comprises a protein with (a) molecular weight of 3,000 to 15,000 and (b) isoelectric point of pI 3.00 to 5.50 (hereinafter referred to as a protein B).

<<Decellularized tissue>>

Decellularized tissues are tissues from which cellular components have been removed from animal-derived tissues, and are mainly composed of extracellular matrix components such as elastin, collagen (type I, IV, etc.), and laminin. It is expected to be less susceptible to rejection at the time of implantation, and to allow the cells of the transplant recipient to settle and reconstruct, and to function as a scaffold for cells.

Conventional methods for obtaining decellularized tissues included in the composition of the present invention can be used. In the present invention, the method of decellularization is not particularly limited as long as the effect of the present invention is obtained. However, there may be mentioned, for example, a high hydrostatic pressure treatment, freezing and thawing method, surfactant treatment, ultrasonic treatment, enzyme treatment, treatment with hypertonic electrolyte solution, physical agitation, hypertonic solution/hypotonic solution method, enzyme treatment with proteolytic enzymes, nucleolytic enzyme, etc., treatment with alcohol solvents, or the like, and a combination of two or more of these methods may be used. In order to efficiently obtain the decellularized tissue composition and to achieve the effect of the present invention, the method by pressure processing is preferable.

Decellularized tissue used for the decellularized tissue composition of the present invention is not particularly limited as long as it is a biological tissue derived from vertebrate animal, but mammalian or avian-derived biological tissue is preferable, because it is less likely to be rejected. Further, mammalian domestic animal, avian domestic animal or human-derived biological tissue is preferable, because it is easier to obtain. Domestic mammals include cattle, horses, camels, llamas, donkeys, yaks, sheep, pigs, goats, deer, alpacas, dogs, Japanese raccoons, weasels, foxes, cats, rabbits, hamsters, guinea pigs, rats, mice, squirrels, raccoons, or the like. Domestic birds include parakeets, parrots, chickens, ducks, turkeys, geese, guinea fowls, pheasants, ostriches, quails, emus or the like. Among these, bovine, pig, rabbit, or human-biological tissue is preferable, due to their availability.

The parts of living tissues with extracellular matrix structures can be used. As such parts, there may be mentioned, for example, a liver, kidney, ureter, bladder, urethra, tongue, tonsil, esophagus, stomach, small intestine, large intestine, anus, pancreas, heart, blood vessel, spleen, lung, brain, bone, spinal cord, cartilage, testis, uterus, fallopian tube, ovary, placenta, cornea, skeletal muscle, tendon, nerve, skin, or the like. The parts of living tissues preferably include cartilage, bone, liver, kidney, heart, pericardium, aorta, skin, small intestinal submucosa, lung, brain, internal thoracic artery, or spinal cord, because of their high tissue regeneration effect, and more preferably include pericardium, internal thoracic artery, liver, cartilage, skin, small intestinal submucosa, or spinal cord.

When the decellularized tissue is obtained by the high hydrostatic pressure treatment, hydrostatic pressure of 50 to 1500 MPa is applied to the living body-derived tissue in the medium. From the viewpoint of sufficient decellularization, the applied hydrostatic pressure is preferably 50 MPa or more. From the viewpoint of not requiring a pressure vessel that can withstand the pressurization, not requiring much energy, and preventing the tissue from being damaged by ice formation when the medium used for the application is a water-based medium, the applied hydrostatic pressure is preferably 1500 MPa or less. The applied hydrostatic pressure of 80 to 1300MPa is more preferable, 90 to 1200MPa is even more preferable, 95 to 1100MPa is even more preferable, 95 to 700MPa is even more preferable, and 400 to 700MPa is most preferable, from the viewpoint of the decellularization effect, bacteriostatic effect and virus inactivation effect, and ease of pressurization.

The media used for hydrostatic pressure application include water, saline solution, injection water, propylene glycol or its aqueous solution, glycerin or its aqueous solution, sugar aqueous solution, or the like. Buffer solutions include acetate buffer, phosphate buffer, citrate buffer, borate buffer, tartrate buffer, Tris buffer, HEPES buffer, or the like. These media may contain surfactants.

The temperature of the high hydrostatic pressure treatment is not particularly limited as long as ice does not form and heat does not damage the tissue. The temperature is preferably 0 to 45°C, more preferably 4 to 37°C, and most preferably 5 to 35°C, because the decellularization process is smoothly performed and the effect on the tissue is minimal. If the duration of the high hydrostatic pressure treatment is too short, the cells are not sufficiently destroyed, and if the duration of the high hydrostatic pressure treatment is too long, energy is wasted. Therefore, the duration to maintain the desired applied pressure in the high hydrostatic pressure treatment is preferably 1 to 120 minutes, more preferably 5 to 60 minutes, and even more preferably 7 to 30 minutes.

The tissue treated with high hydrostatic pressure is preferably treated with nucleolytic enzymes. The nucleolytic enzyme removes nucleic acid components from the hydrostatically treated living tissues, and is not particularly limited to, for example, DNase (such as DNase I, DNase II) derived from pancreas, spleen, or E. coli.

The nucleolytic enzyme can be added to the medium (such as water, saline solution, injection solution, or buffer solution) used for the above high hydrostatic pressure treatment, to make the enzyme work. The amount of enzyme to be added depends on the type of enzyme and the definition of the number of units (U), but can be set appropriately by a person skilled in the art. In the case of DNase I, for example, 50~200 U/mL may be used. The treatment temperature also depends on the nucleolytic enzyme to be used, but for example, may be set at 1°C to 40°C. The treatment time is not particularly limited, but for example, may be set to 1 to 120 hours (preferably 1 to 96 hours, more preferably 1 to 48 hours). In case of low temperatures, the treatment may be long, and in case of high temperatures, the treatment time may be short.

The tissue treated with high hydrostatic pressure is washed with a washing liquid. The washing liquid may be the same as or different from the medium used for the high hydrostatic pressure treatment. The washing liquids preferably contain organic solvents or chelating agents. Organic solvents can improve the removal efficiency of lipids, and chelating agents can inactivate calcium and magnesium ions in the decellularized tissue, thereby preventing calcification when the particulate decellularized tissue of the present invention is applied to diseased areas. As organic solvents, water-soluble organic solvents are preferable because they are effective in removing lipids, and ethanol, isopropanol, acetone, and dimethyl sulfoxide are preferable. As chelating agents, there may be mentioned iminocarboxylic acid chelating agents, such as ethylenediaminetetraacetic acid (EDTA), nitrilotriacetic acid (NTA), diethylenetriamine pentaacetic acid (DTPA), hydroxyethylenediaminetriacetic acid (HEDTA), triethylenetetramine hexaacetic acid (TTHA), 1,3-propanediaminetetraacetic acid (PDTA), and 1,3-diamino-2-hydroxypropane tetraacetic acid (DPTA-OH), hydroxyethyl iminodiacetic acid (HIDA), dihydroxyethylglycine (DHEG), glycol ether diamine tetraacetic acid (GEDTA), dicarboxymethylglutamic acid (CMGA), 3-hydroxy-2, 2'-iminodisuccinic acid (HIDA), dicarboxymethyl aspartate (ASDA) or their salts; hydroxycarboxylic chelating agents, such as citric acid, tartaric acid, malic acid and lactic acid, or their salts. The salts of these chelating agents include sodium or potassium salts.

The washing temperature is not particularly limited as long as there is no heat damage to the tissue. The washing temperature is preferably 0 to 45°C, more preferably 1 to 40°C, and most preferably 2 to 35°C, since the washing property is good and there is little effect on the tissue. When washing, the washing liquid may be shaken or stirred as necessary.

When the decellularized tissue is obtained by the freezing and thawing method, the living body-derived tissue is frozen at a temperature of -80 to -20°C (preferably -80 to -40°C) for 1 to 48 hours (preferably 10 to 30 hours), and then thawed at a temperature of 20 to 37°C. The above process is repeated once or more than twice (preferably 2 to 5 times). Then, the tissue is preferably treated with nucleolytic enzymes in the same way as the high hydrostatic pressure treatment. The cells in the frozen and thawed living tissues are destroyed, and these cells are removed by the washing liquid. The washing method is the same as that in the high hydrostatic pressure treatment.

When the decellularized tissue is obtained by the surfactant treatment, the living body-derived tissue is shaken with a surfactant solution (such as 0.25 mass% sodium dodecyl sulfate (SDS) solution) for 1 to 48 hours (preferably 12 to 36 hours) at 2 to 10°C (preferably 4°C). Further, the living body-derived tissue may be shaken with a different surfactant solution (such as 0.5% mass% Triton X (polyoxyethylene octylphenyl ether) solution) for 1 to 48 hours (preferably 12 to 36 hours) at 2 to 10°C (preferably 4°C). Then, it is preferably washed in the same way as the high hydrostatic pressure treatment.

Surfactants is not limited to, for example, but include sodium dodecyl sulfate, alkyl sulfonate, alkyl sulfate ester, polyoxyethylene alkyl sulfate ester, alpha-sulfo fatty acid ester, polyoxyethylene alkyl ether, and polyoxyethylene alkyl phenyl ether (such as polyoxyethylene octyl phenyl ether), and alkyl (poly)glycoside.

When the decellularized tissue is obtained by the ultrasonic treatment, the living body-derived tissue is treated with ultrasonic waves (for example, intensity: 10W/cm$^2$, frequency: 10 kHz, duration: 2minutes), for example, in a saline solution. Then, the tissues are preferably shaken with a surfactant solution (such as 1 mass% Triton X (polyoxyethylene octylphenyl ether) solution) at 2 to 10°C (preferably 4°C) for 1 to 120 hours (preferably 12 to 120 hours). Then, the tissue is preferably treated with nucleolytic enzymes in the same way as the high hydrostatic pressure treatment. Then, it is preferably washed in the same way as the high hydrostatic pressure treatment.

The obtained decellularized tissue is preferably freeze-dried, although this is not a limitation. Freeze-drying can be omitted depending on the site of the living tissue. In addition, the decellularized tissues may be sterilized by gamma irradiation, UV irradiation, or the like, and the sterilization by gamma irradiation is preferable.

From the viewpoint of exhibiting the effect of the present invention, the amount of the decellularized tissue is preferably 90.0 to 100.0 % by weight, more preferably 95.0 to 100.0 % by weight, even more preferably 96.0 to 100.0 % by weight, and most preferably 97.0 to 100.0 % by weight with respect to the decellularized tissue composition.

(Decellularized DNA ratio (DNA content))

In general, it is preferable to reduce the DNA content in decellularized tissue compositions. In the decellularized tissue composition of the present invention, the DNA content per dry mass is preferably 0.0300 % by weight or less with respect to the decellularized tissue composition. Whereby the occurrence of rejection can be prevented when it is used for regenerative medicine. From the viewpoints of the cell attraction effect and cell differentiation induction effect, the decellularized DNA ratio in the decellularized tissue composition of the present invention is preferably 0.0250 % by weight or less, more preferably 0.0200 % by weight or less, even more preferably 0.0150% by weight or less, and most preferably 0.0120% by weight or less. The decellularized DNA ratio of 0.0150 mass% or less is preferable in that it particularly facilitates cell adhesiveness to the decellularized tissue composition.

A lower limit of the decellularized DNA ratio is not particularly limited, but the lower is better. From the viewpoint of feasibility, it is preferably 0.0001% by weight or more, and even more preferably 0.0002 % by weight or more.

The DNA content can be measured by the PicoGreen method. A dried specimen of the decellularized tissue composition (hereinafter referred to as "sample") is immersed in proteolytic enzyme solution to dissolve the same. Then, it is treated with phenol/chloroform to remove proteins, and DNA is recovered. The recovered DNA is fluorescently stained with PicoGreen (Life Technologies) and the DNA is quantified by measuring the fluorescence intensity, and the DNA content (mass) of the sample is calculated. For quantification, a calibration curve prepared using the standard DNA provided with Picogreen is used. From the dry mass and DNA content of the sample, the DNA ratio is calculated according to the following formula.

(DNA content per dry mass (hereinafter referred to as decellularized DNA ratio)) = (DNA content of dried specimen of decellularized tissue composition)/(mass of dried specimen of decellularized

<< Protein >>

The decellularized tissue composition of the present invention comprises a protein A with (a) molecular weight of 30,000 to 70,000 and (b) isoelectric point of pI6.00 to 9.00. The decellularized tissue composition of the present invention may further comprises a protein with (a) molecular weight of 3,000 to 15,000 and (b) isoelectric point of pI3.00 to 5.50. Whereby the effect of the present invention is further exhibited.

The molecular weight and isoelectric point (pI) of the above proteins can be determined, for example, by two-dimensional electrophoresis of the proteins and the above proteins can be identified by mass spectrometry.

In two-dimensional electrophoresis, either isoelectric point separation or molecular weight separation can be performed first in the first dimension. However, from the viewpoint of increasing the accuracy of measurement, it is preferable to perform isoelectric point separation in the first dimension and molecular weight separation in the second dimension. Two-dimensional electrophoresis can be performed according to the conventional method, and commercially available kits and apparatuses can be used. For example, isoelectric electrophoresis is performed using capillary gels or strip gels as separation media, and after the gel swimming is completed, molecular weight separation can be performed by electrophoresis in the direction perpendicular to the direction of expansion of isoelectric electrophoresis using a planar gel (such as SDS-polyacrylamide gel). The presence or absence of protein, molecular weight, and isoelectric point can be confirmed by staining the gel after two-dimensional electrophoresis according to the conventional method.

The molecular weight of the protein A is preferably 30,000 to 70,000, more preferably 30,000 to 50,000, and even more preferably 30,000 to 40,000, from the viewpoint of exhibiting the effect of the present invention. The isoelectric point (pI) of protein A is preferably 6.00 to 9.00, more preferably 6.00 to 8.50, and even more preferably 6.50 to 8.50.

The protein A is preferably an annexin. Annexins are proteins with a curved core domain consisting of four or eight $\alpha$-helical structures, so-called annexin repeats (about 70 amino acid residues). The amino-terminal domain is unique to each annexin (11 to 196 residues), while the carboxy-terminal domain is well conserved among annexins. Calcium binding sites and phospholipid binding sites are located on the carboxy-terminal domain. The molecular weight of annexin VI with eight annexin repeats is about 66kd.

The origin of annexins contained in the decellularized tissue composition is not limited as long as they are derived from eukaryotes, but mammalian or avian-derived annexins are preferable. Mammals include cattle, horses, camels, llamas, donkeys, yaks, sheep, pigs, goats, deer, alpacas, dogs, Japanese raccoons, weasels, foxes, cats, rabbits, hamsters, guinea pigs, rats, mice, squirrels, raccoons, or the like. Birds include parakeets, parrots, chickens, ducks, turkeys, geese, guinea fowls, pheasants, ostriches, quails, emus or the like.

In vertebrates, there are 12 kinds of annexins, annexins A1 to A11 and A13, which have binding sites for $Ca^{2+}$ and phospholipids. For example, S100A10 which has a C-terminal lysine, binds to the concave N-terminal region of annexin A2, and it becomes the binding site of tissue-type plasminogen activator (tPA) and plasminogen.

The annexins include annexins with different modifications. That is to say, annexins that are phosphorylated, glycosylated, ubiquitinated, nitrosylated, methylated, or acetylated, or annexins without these modifications are included. For example, the annexin A2 described in the Examples are three types of annexin A2 with different phosphorylation, but all of them can exhibit the effect of the present invention.

Furthermore, the decellularized tissue composition of the present invention can include, without limitation, variants of annexin, as long as the effects of the present invention is obtained. The annexin variant include, for example

(1) a polypeptide containing an amino acid sequence in which one or more amino acids (preferably 1 to 10, more preferably 1 to 7, and even more preferably 1 to 5) in total, for example, one to several amino acids in total, are deleted, substituted, inserted, and/or added at one or more locations in the amino acid sequence of annexin (for example, the amino acid sequence represented by SEQ ID No. 1), and exhibiting annexin activity, or
(2) a polypeptide having an amino acid sequence having 90% or more homology with the amino acid sequence of annexin (for example, the amino acid sequence represented by SEQ ID No. 1), and exhibiting annexin activity.

The annexin activity includes, for example, the ability to bind $Ca^{2+}$ and phospholipids. Alternatively, as the decellularized tissue composition of the present invention, the cell adhesiveness is improved, compared to that without the modified form.

(Amino acid sequence in which one or more amino acids are deleted, substituted, inserted, and/or added)

The variant may be a polypeptide consisting of an amino acid sequence in which one or more amino acids are deleted, substituted, inserted, and/or added in the amino acid sequence of annexin (for example SEQ ID No. 1). The polypeptide of variant has a binding ability to $Ca^{2+}$ and phospholipids. In other words, a polypeptide that does not exhibit binding ability to $Ca^{2+}$ and phospholipids is not included in the polypeptide of variant. In this specification, the wording "amino acid sequence in which one or more amino acids are deleted, substituted, inserted, and/or added" means that the amino acid sequence has been modified by amino acid substitutions or the like. The number of amino acid modifications may be 1 to 30, 1 to 20, 1 to 15, 1 to 10, preferably 1 to 8, more preferably 1 to 6, even more preferably 1 to 5, most preferably 1 to 2. Preferably, examples of amino acid sequences of the variants that can be used in the present invention may be amino acid sequences having one or more (preferably, 1, 2, 3 or 4) conservative substitutions of its amino acids.

(Amino acid sequence having 90% or more identity to amino acid sequence)

The variant may be a polypeptide consisting of an amino acid sequence that has 90% or more identity with the amino acid sequence of annexin (for example SEQ ID No. 1). The polypeptide of variant has a binding ability to $Ca^{2+}$ and phospholipids. In other words, a polypeptide that does not exhibit binding ability to $Ca^{2+}$ and phospholipids is not included in the polypeptide of variant. The polypeptide of variant has a binding ability to $Ca^{2+}$ and phospholipids. In other words, polypeptide that does not exhibit binding ability to $Ca^{2+}$ and phospholipids is not included in the polypeptide of variant. The polypeptide comprises an amino acid sequence having the identity is more preferably 95% or more, more preferably 96% or more, more preferably 97% or more, more preferably 98% or more, more preferably 99% or more, and exhibits the binding ability to $Ca^{2+}$ and phospholipids.

The "amino acid sequence in which in which one or more amino acids are deleted, substituted, inserted, and/or added" or "amino acid sequence having 90% or more identity to amino acid sequence" in the amino acid sequence of the annexin (for example, SEQ ID No. 1) is an amino acid sequence of an annexin (for example, SEQ ID No. 1) is substituted. However, The substitutions in the amino acid sequence are conservative substitutions that maintain the function of the annexin used in the present invention. In other words, the "conservative substitution" means a substitution that does not cause loss of the excellent effect of annexin. That is to say, it is a substitution that maintains the binding ability of annexin to $Ca^{2+}$ and phospholipids even in the case of insertion, substitution, deletion, or addition. Specifically, it means the replacement of an amino acid residue with another chemically similar amino acid residue. For example, as the conservative substitution, there may be mentioned a substitution of a hydrophobic residue for another hydrophobic residue, or a substitution of a polar residue for another polar residue having the same charge. Amino acids which have similar chemical properties and can be conservatively substituted with each other are known to those skilled in the art. More particularly, as nonpolar (hydrophobic) amino acids, there may be mentioned, for example, alanine, valine, isoleucine, leucine, proline, tryptophan, phenylalanine, or methionine. As polar (neutral) amino acids, there may be mentioned, for example, glycine, serine, threonine, tyrosine, glutamine, asparagine, or cysteine. As basic amino acids having a positive charge, there may be mentioned, for example, arginine, histidine, or lysine. As acidic amino acids having a negative charge, there may be mentioned, for example, aspartic acid or glutamic acid.

The amount of protein A is preferably 0.01 to 5.0% by weight, more preferably 0.03 to 4.5 % by weight, further more preferably 0.05 to 4.0 % by weight, even more preferably 0.1 to 3.5 % by weight, even more preferably 0.2 to 3.3 % by weight, most preferably 0.3 to 3.0% by weight with respect to the decellularized tissue composition. Whereby the effect of the present invention is exhibited.

The molecular weight of the protein B is preferably 3,000 to 15,000, more preferably 3,000 to 15,000, and even more preferably 7,000 to 13,000, from the viewpoint of exhibiting the effect of the present invention. The isoelectric point (pI) of the protein is preferably 3.00 to 5.50, more preferably 3.40 to 5.30, and even more preferably 3.80 to 5.20.

The amount of protein B is preferably 0.001 to 3.0% by weight, more preferably 0.003 to 2.0% by weight, further more preferably 0.005 to 1.5 % by weight, even more preferably 0.008 to 1.0 % by weight, even more preferably 0.009 to 0.5 % by weight, most preferably 0.01 to 0.1% by weight with respect to the decellularized tissue composition. Whereby the effect of the present invention is exhibited.

The decellularized tissue composition of the present invention preferably contains protein A and protein B in a mass ratio of A:B is 5:1 to 150:1, more preferably in a mass ratio of A:B is 8:1 to 110:1, and even more preferably in a mass ratio of A:B is 10:1 to 50:1. Whereby the effect of the present invention is effectively exhibited.

The protein B includes preferably a fragment of fibromodulin. Fibromodulin is a protein that mainly binds to fibrous collagen and is thought to play a role in binding collagen fibers together. It is known that the density of collagen fibers increases when the expression of fibromodulin is upregulated. Fibromodulin is a protein (SEQ ID No. 2) consisting of 375 amino acids with a molecular weight of 43,000 and a pI of 5.6.

Protein B is not particularly limited, but is a fragment of fibromodulin, and is a fragment (SEQ ID No. 3) consisting of 90 amino acids at the C-terminus, which is estimated from the molecular weight. The molecular weight of the fibromodulin fragment is 10,000 and a pI thereof is 4.65.

The origin of fibromodulin fragments contained in the decellularized tissue composition is not limited as long as they are derived from eukaryotes, as is the case with annexins.

The decellularized tissue composition of the present invention can include, without limitation, variants of fibromodulin fragments, as long as the effects of the present invention is obtained. The fibromodulin fragment variants include, for example

(1) a polypeptide containing an amino acid sequence in which one or more amino acids (preferably 1 to 10, more preferably 1 to 7, and even more preferably 1 to 5) in total, for example, one to several amino acids in total, are deleted, substituted, inserted, and/or added at one or more locations in the amino acid sequence of fibromodulin fragment (for example, the amino acid sequence represented by SEQ ID No. 3), and exhibiting fibromodulin fragment activity, or

(2) a polypeptide having an amino acid sequence having 90% or more homology with the amino acid sequence of fibromodulin fragment (for example, the amino acid sequence represented by SEQ ID No. 3), and exhibiting fibromodulin fragment activity.

As the fibromodulin fragment activity, the cell adhesiveness is improved, compared to that without the modified form.

The fibromodulin fragment variants exhibit the same amino acid mutations, identity, and conservative substitutions as the above annexin variants.

The fibromodulin fragments are considered to be generated from fibromodulin by gamma irradiation of the decellularized tissue compositions. The intensity of gamma irradiation is not particularly limited as long as the effects of the present invention is obtained, but is 10 to 50 kGy, preferably 15 to 35 kGy, and more preferably 20 to 30 kGy. The fibromodulin fragments can be produced by the above range.

<<Function>>

The reasons why the decellularized tissue compositions of the present invention exhibit excellent strength and cell adhesiveness are not completely analyzed, but it can be estimated as follows. However, the present invention is not limited by the following explanations.

Protein A (for example, annexin) contained in the decellularized tissue composition of the present invention has four or eight $\alpha$-helical structures (annexin repeats) and binds to $Ca^{2+}$ and phospholipids. The binding ability of annexin to $Ca^{2+}$ and phospholipids is considered to be related to the cell adhesion ability, although this is not a limitation. It is also considered that annexin effectively acts on the strength of decellularized tissues.

Further, it is considered that protein B (for example, fibromodulin fragment) in the decellularized tissue composition is produced by the cleavage of fibromodulin by gamma irradiation. Fibromodulin has a role of stabilizing collagen fibers, and it is considered that the presence of sufficient fibromodulin suppresses cellular collagen synthesis. On the other hand, when fibromodulin is destroyed, it is presumed that the inhibition of collagen neogenesis is lost in cells in contact with this fragment. Therefore, it is assumed that cellular collagenesis occurs, cell functions are activated, and the number of cells adhering to the decellularized tissue composition increases.

The decellularized tissue composition of the present invention can be produced by the method for obtaining decellularized tissue. In this case, in order to efficiently obtain the decellularized tissue composition, and in order to remarkably achieve the effect of the present invention, it is preferable to use the method of high hydrostatic pressure treatment.

The decellularized tissue composition of the present invention can be obtained by externally adding the above proteins to the decellularized tissue obtained by any method.

EXAMPLES

The present invention will now be further illustrated by, but is by no means limited to, the following Examples.

<<Example 1>>

In this example, a decellularized tissue composition was prepared using a bovine pericardium by high hydrostatic pressure treatment.

The bovine pericardium was cut open to form a sheet, and the fat was removed entirely (hereinafter, this sheet of bovine pericardium is referred to as "pericardial sheet"). The pericardial sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried to obtain the decellularized tissue composition of Example 1.

<<Example 2>>

In this example, a decellularized material was prepared using a bovine pericardium by surfactant treatment, and annexin A2 was added to obtain the decellularized tissue composition.

The pericardial sheet was sterilized by immersion in a 0.1% (v/v) peracetic acid solution prepared to contain 4% ethanol, at 4°C for 2 hours, and then washed with injection water at 4°C for 1 hour. Then, it was shaken in 0.25 % by weight of sodium dodecyl sulfate (SDS) solution (10 mM Tris, pH 8.0) for 24 hours at 4°C, and then shaken in 0.5% by weight of Triton-X (polyoxyethylene octylphenyl ether: hereinafter referred to as TX) solution (10 mM Tris, pH 8.0) for 24 hours at 4°C. After washing with 10 L of injection water at 4°C, it was shaken in phosphate buffer solution (PBS, 0.01 M, pH 7.4) at 4°C for 1 hour. After freeze-drying, annexin A2 was added at 0.3 % by weight per weight of decellularized tissue composition to obtain the decellularized tissue composition of Example 2.

<<Example 3>>

In this example, a decellularized material was prepared using a bovine pericardium by freezing and thawing treatment, and annexin A2 was added to obtain the decellularized tissue composition.

The pericardium sheet was frozen with dry ice, stored for 20 hours in a cold box filled with dry ice (at approximately -78°C), and then thawed at 25°C. Then, the pericardium sheet was repeatedly frozen and thawed four times. The resulting pericardial sheet was treated with DNase I (125 U/mL) as a nucleolytic enzyme and shaken at 4°C for 96 hours. Then, the sheet was shaken in 80% ethanol at 4°C for 96 hours, followed by shaking in injection water at 4°C for 2 hours. After freeze-drying, annexin A2 was added at 0.3 % by weight per weight of decellularized tissue composition to obtain the decellularized tissue composition of Example 3.

<<Example 4>>

In this example, a decellularized material was prepared using a bovine pericardium by ultrasonic treatment, and annexin A2 was added to obtain the decellularized tissue composition.

The pericardium sheet was subjected to ultrasonic treatment (intensity: 10 W/cm$^2$, frequency: 10 kHz, duration: 2 min) in physiological saline solution. The ultrasonically treated bovine pericardium was shaken in 1 % by weight of TX solution (10 mM Tris, pH 8.0) at 4°C for 96 hours. The TX-treated pericardial sheet was treated with DNase I (125 U/mL) as a nucleolytic enzyme and shaken at 4°C for 96 hours. Then, the sheet was shaken in 80% ethanol at 4°C for 72 hours, followed by shaking in saline solution at 4°C for 2 hours. After freeze-drying, annexin A2 was added at 0.3 % by weight per weight of decellularized tissue composition to obtain the decellularized tissue composition of Example 4.

<<Example 5>>

In this example, a decellularized material was prepared using a bovine pericardium by high hydrostatic pressure treatment, and annexin A2 was added to obtain the decellularized tissue composition.

The bovine pericardium was cut open to form a sheet, and the fat was removed entirely to obtain a pericardial sheet. The pericardial sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried to obtain a decellularized bovine pericardial membrane. Annexin A2 was added thereto at 0.3 % by weight per weight of decellularized bovine pericardial membrane to obtain the decellularized tissue composition of Example 5.

<<Example 6>>

In this example, a decellularized material was prepared using a bovine pericardium by surfactant treatment, and annexin A2 was added to obtain the decellularized tissue composition.

The pericardial sheet was sterilized by immersion in a 0.1% (v/v) peracetic acid solution prepared to contain 4% ethanol, at 4°C for 2 hours, and then washed with injection water at 4°C for 1 hour. Then, it was shaken in 0.25 % by weight of SDS solution (10 mM Tris, pH 8.0) for 24 hours at 4°C, and then shaken in 0.5% by weight of TX solution (10 mM Tris, pH 8.0) for 24 hours at 4°C. After washing with 10 L of injection water at 4°C, it was shaken in phosphate buffer solution (PBS, 0.01 M, pH 7.4) at 4°C for 1 hour. After freeze-drying, annexin A2 was added at 1.0 % by weight per weight of decellularized tissue composition to obtain the decellularized tissue composition of Example 6.

<<Example 7>>

In this example, a decellularized material was prepared using a bovine pericardium by freezing and thawing treatment, and annexin A2 was added to obtain the decellularized tissue composition.

The pericardium sheet was frozen with dry ice, stored for 20 hours in a cold box filled with dry ice (at approximately -78°C), and then thawed at 25°C. Then, the pericardium sheet was repeatedly frozen and thawed four times. The resulting pericardial sheet was treated with DNase I (125 U/mL) as a nucleolytic enzyme and shaken at 4°C for 96 hours. Then, the sheet was shaken in 80% ethanol at 4°C for 96 hours, followed by shaking in injection water at 4°C for 2 hours. After freeze-drying, annexin A2 was added at 1.0 % by weight per weight of decellularized tissue composition to obtain the decellularized tissue composition of Example 7.

<<Example 8>>

In this example, a decellularized material was prepared using a bovine pericardium by ultrasonic treatment, and annexin A2 was added to obtain the decellularized tissue composition.
The pericardium sheet was subjected to ultrasonic treatment (intensity: 10 W/cm$^2$, frequency: 10 kHz, duration: 2 min) in physiological saline solution. The ultrasonically treated bovine pericardium was shaken in 1 % by weight of TX solution (10 mM Tris, pH 8.0) at 4°C for 96 hours. The TX-treated pericardial sheet was treated with DNase I (125 U/mL) as a nucleolytic enzyme and shaken at 4°C for 96 hours. Then, the sheet was shaken in 80% ethanol at 4°C for 72 hours, followed by shaking in saline solution at 4°C for 2 hours. After freeze-drying, annexin A2 was added at 1.0 % by weight per weight of decellularized tissue composition to obtain the decellularized tissue composition of Example 8.

<<Comparative Example 1>>

In this comparative example, a decellularized material was prepared using a bovine pericardium by surfactant treatment.
The pericardial sheet was sterilized by immersion in a 0.1% (v/v) peracetic acid solution prepared to contain 4% ethanol, at 4°C for 2 hours, and then washed with injection water at 4°C for 1 hour. Then, it was shaken in 0.25 % by weight of SDS solution (10 mM Tris, pH 8.0) for 24 hours at 4°C, and then shaken in 0.5% by weight of TX solution (10 mM Tris, pH 8.0) for 24 hours at 4°C. After washing with 10 L of injection water at 4°C, it was shaken in phosphate buffer solution (PBS, 0.01 M, pH 7.4) at 4°C for 1 hour. It was freeze-dried to obtain a decellularized bovine pericardial membrane (decellularized material) of Comparative Example 1.

<<Comparative Example 2>>

In this comparative example, a decellularized material was prepared using a bovine pericardium by freezing and thawing treatment.
The pericardium sheet was frozen with dry ice, stored for 20 hours in a cold box filled with dry ice (at approximately -78°C), and then thawed at 25°C. Then, the pericardium sheet was repeatedly frozen and thawed four times. The resulting pericardial sheet was treated with DNase I (125 U/mL) as a nucleolytic enzyme and shaken at 4°C for 96 hours. Then, the sheet was shaken in 80% ethanol at 4°C for 96 hours, followed by shaking in injection water at 4°C for 2 hours. It was freeze-dried to obtain a decellularized bovine pericardial membrane (decellularized material) of Comparative Example 2.

<<Comparative Example 3>>

In this comparative example, a decellularized material was prepared using a bovine pericardium by ultrasonic treatment.
The pericardium sheet was subjected to ultrasonic treatment (intensity: 10 W/cm$^2$, frequency: 10 kHz, duration: 2 min) in physiological saline solution. The ultrasonically treated bovine pericardium was shaken in 1 % by weight of TX solution (10 mM Tris, pH 8.0) at 4°C for 96 hours. The TX-treated pericardial sheet was treated with DNase I (125 U/mL) as a nucleolytic enzyme and shaken at 4°C for 96 hours. Then, the sheet was shaken in 80% ethanol at 4°C for 72 hours, followed by shaking in saline solution at 4°C for 2 hours. It was freeze-dried to obtain a decellularized bovine pericardial membrane (decellularized material) of Comparative Example 3.

<<Cell adhesion test>>

The decellularized tissue compositions or decellularized materials obtained in the examples and comparative examples were cut into 4 mm-diameter disks using autoclaved scissors and tweezers and placed in cell inserts for 96-well plates. 175 μL or 15 μL of DMEM medium was added to the lower side or to the upper side of the cell insert respectively, and the cell insert was incubated at 37°C for at least 24 hours to acclimate the decellularized tissue composition or decellularized material. After removal of the DMEM medium, normal human dermal fibroblasts (NHDF) were seeded ($7.0 \times 10^4$ cells/cm$^2$) and cultured at 37°C for 3 hours. After washing and removing the cells not adhering to the specimens using DPBS (Dulbecco's Phosphate-Buffered Saline), the specimens were stained with Wst-8 (Dojindo Laboratories) and the absorbance at 450 nm was measured. A calibration curve was separately prepared from the absorbance of NHDF stained by Wst-8. The number of NHDF adhering to the specimens was calculated from the absorbance of the specimens and the calibration curve.

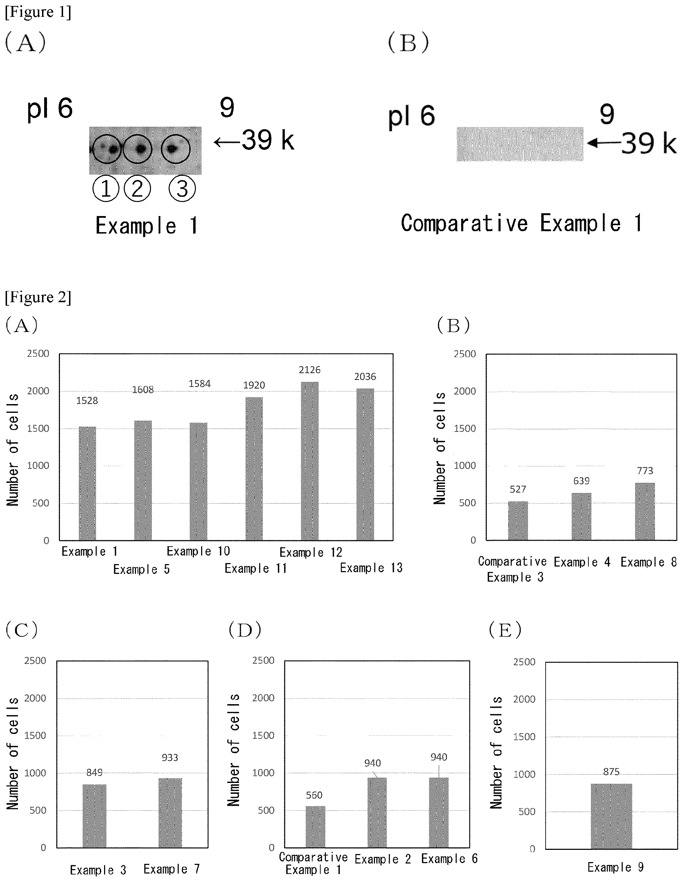

The results of cell adhesion tests are shown in Figure 2. The result of Comparative Example 2 was similar to that of Comparative Example 1. Compared to Comparative Example 1, the number of cells adhering to the specimens in Examples 1 to 8 was higher, indicating that the cell attraction effects of the specimens were better. In particular, Examples 1 and 5 exhibit good cell attraction effects.

<<Example 9>>

In this example, a decellularized tissue composition was prepared using a swine liver by high hydrostatic pressure treatment.

The swine liver was cut open to form a sheet, and the fat was removed entirely (hereinafter, this sheet of swine liver is referred to as "liver sheet"). The liver sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated liver sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried to obtain the decellularized tissue composition of Example 9.

<<Example 10>>

In this example, a decellularized material was prepared using a bovine pericardium by high hydrostatic pressure treatment, and annexin A2 was added to obtain the decellularized tissue composition.

The bovine pericardium was cut open to form a sheet, and the fat was removed entirely to obtain a pericardial sheet. The pericardial sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried to obtain a decellularized bovine pericardial membrane. Annexin A2 was added thereto at 3.0 % by weight per weight of decellularized tissue composition to obtain the decellularized tissue composition of Example 10.

<<Example 11>>

In this example, a decellularized tissue composition was prepared using a bovine pericardium by high hydrostatic pressure treatment.

The bovine pericardium was cut open to form a sheet, and the fat was removed entirely (hereinafter, this sheet of bovine pericardium is referred to as "pericardial sheet"). The pericardial sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried, and then gamma-irradiated (25kGy) to obtain the decellularized tissue composition of Example 11.

<<Example 12>>

In this example, a decellularized material was prepared using a bovine pericardium by high hydrostatic pressure treatment, and annexin A2 was added to obtain the decellularized tissue composition.

The bovine pericardium was cut open to form a sheet, and the fat was removed entirely to obtain a pericardial sheet. The pericardial sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried, and then gamma-irradiated (25kGy) to obtain a decellularized bovine pericardial membrane. Annexin A2 was added thereto at 1.0 % by weight per weight of decellularized bovine pericardial membrane to obtain the decellularized tissue composition of Example 12.

<<Example 13>>

In this example, a decellularized material was prepared using a bovine pericardium by high hydrostatic pressure treatment, and annexin A2 was added to obtain the decellularized tissue composition.

The bovine pericardium was cut open to form a sheet, and the fat was removed entirely to obtain a pericardial sheet. The pericardial sheet was placed in a polyethylene bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), shaken at 4°C for 18 h 5 min or more, then treated in 80% ethanol at 4°C for at least 1 hour, and finally washed with 4 L of injection water at 4°C. Then, the sheet was freeze-dried, and then gamma-irradiated (25kG) to obtain a decellularized bovine pericardial membrane. Annexin A2 was added thereto at 3.0 % by weight per weight of decellularized bovine pericardial membrane to obtain the decellularized tissue composition of Example 13.

The above cell adhesion tests were performed on Examples 9 to 13. The results are shown in Figure 2. It was found that the number of adhered cells adhering of Examples 9~13 was larger than that of Comparative Example 1, and that the cell attraction effect was better. In particular, Example 11 exhibited good cell attraction effect. Further, in Example 9, it was found that good result was obtained even when swine liver was used.

<< Strength test >>

(Tensile test)

Tensile tests of decellularized tissue compositions or decellularized materials obtained in Examples 1 and 11 and Comparative Example 1 were performed as follows.

(1) Collection and preparation of test specimens

The freeze-dried decellularized tissue compositions or decellularized materials were immersed in physiological saline solution for at least 15 minutes. Dumbbell-shaped No. 8 specimens as described in ISO 37 was taken from the swollen decellularized tissue compositions or decellularized materials.

(2) Measurement of specimen

The thickness of the parallel portions of the dumbbell specimens was measured using a 3D one-shot geometry measuring device (VR-3200, Keyence). The width (mm) of the specimen was taken as the length of the cut surface tube (40 mm) of the punching blade of the parallel section. The cross-sectional area A (mm$^2$) of the specimen was calculated from the thickness and width of the specimen using the following formula

$$A = t \times w$$

(A: specimen cross-sectional area (mm$^2$), t: specimen thickness (mm), w: specimen width (mm))

(3) Test procedure

Tensile tests were conducted in accordance with ISO 37 as follows. The specimens were mounted on a mechanical testing machine (MCT2150, manufactured by AND) so that both ends of the specimens were held contrastingly in order to distribute the tensile force uniformly across the cross section. The distance Lb (mm) between the marked lines for the operation of the testing machine was measured. The speed of the gripping fixture was 200 mm/min. The data of the specimens that broke outside between the marked lines were discarded, and the test was repeated with additional specimens. Specimens were tested until three correct measurements were obtained. From the measured values, the breaking strength [MPa] and elastic modulus [MPa] (at 10% elongation and at maximum) were calculated using the following formulas.

(4) Results of Calculation

<Breaking strength>

The breaking strength (MPa(N/mm$^2$)) is calculated by the following formula.

$$\text{Breaking strength (MPa)}=\text{Maximum load (N)}/\text{Section of specimen (mm2)}$$

<Elastic modulus>

The elastic modulus (stress/strain) is calculated by the following formula.

$$\text{Elastic modulus (MPa)}=\text{Load (MPa(N/mm}^2\text{))}/\text{Strain ((L-L}_0\text{)/L}_0\text{)}$$

(Suture strength test)

Suture strength tests of decellularized tissue compositions or decellularized materials obtained in the Examples and Comparative Examples were performed as follows.

(1) Collection and preparation of test specimens

The freeze-dried decellularized tissue compositions or decellularized materials were immersed in physiological saline solution for at least 15 minutes. The specimens were taken from the swollen decellularized tissue composition or decellularized material to be 20 mm × 15 mm. A hole of 6mm diameter was made at 2mm from the top of the long edge. A thread was threaded through the hole, to prepare a test specimen.

(2) Test procedure

One end of the specimen was attached to the gripping fixture of a mechanical testing machine (MCT2150, manufactured by AND), and the thread was placed on the hook-type fixture. The speed of the gripping fixture was 200 mm/min. Specimens were tested until three correct measurements were obtained. The measured value was calculated as the maximum load at break [N].

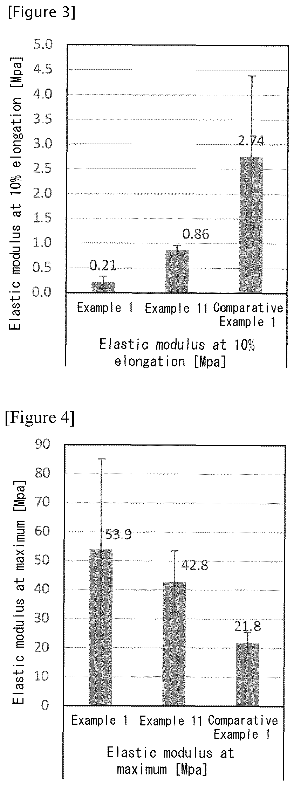

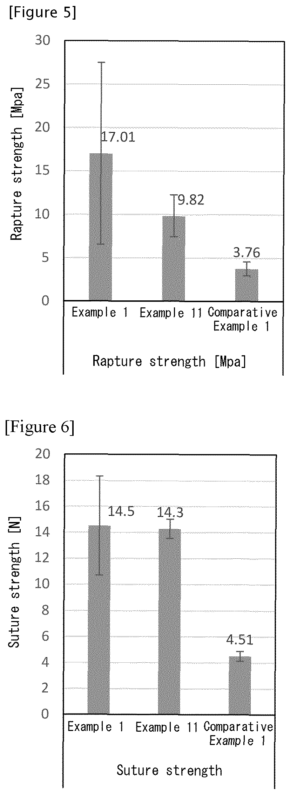

The test results of elastic modulus at 10% elongation, maximum modulus, breaking strength, and suture strength are shown in Figures 3 to 6, respectively. Compared with Comparative Example 1, Examples 1 and 11 require less force for 10% elongation. That is to say, they are softer and have better handling properties. In addition, the maximum modulus of Examples 1 and 11 were larger than that of Comparative Example 1, and were close to the elastic modulus of human pericardium, 49.62±3.22 (MPa, see Interactive Cardio Vascular and Thoracic Surgery22 (2016) 72-84). This allows the implantation of the device in a patient to easily blend with the patient's tissue and maintain the stability of the structure against pulse and pulsation. In particular, Example 1 exhibited a maximum modulus of 53.9 Mpa. In addition, the breaking strengths of Examples 1 and 11 were higher than that of Comparative Example 1. That is to say, they are stronger and tougher than that of Comparative Example 1. Moreover, Examples 1 and 11 have higher suture strength than that of Comparative example 1. That is to say, it is found that they have higher resistance to threading and higher suture strength when used in surgery. Thus, it is found that Examples 1 and 11 have better handling properties and are stronger than that of Comparative Example 1.

<<Protein analysis >>

The compositional differences of the decellularized tissue composition of Example 1 and the decellularized material of Comparative Example 1 were analyzed by two-dimensional electrophoresis.

20 mg of the decellularized tissue composition of Example 1 or the decellularized material of Comparative Example 1, was weighted and put into two microtubes (10 mg per tube), respectively. Protein extraction buffer 1 (containing urea) was added to one tube, and protein extraction buffer 2 (containing urea and surfactant) to the other tube, and shaken at room temperature. Then they were centrifuged, and the supernatants were collected. After the buffer replacement by ultrafiltration unit (Amicon Ultra-4, Ultracel 3k, Millipore), a buffer containing urea was added, concentrated by centrifugation, and the protein was purified. Proteins extracted with protein extraction buffers 1 and 2 were quantitated respectively (Pierce 660nm Protein Assay, Thermo Scientific). Proteins extracted with protein extraction buffers 1 and 2 were mixed. From 20 mg of decellularized tissue composition of Example or 20 mg of decellularized material of Comparative Example, 4.91 mg and 6.06 mg of protein were recovered, respectively. From the sample solution of Example or Comparative Example, 200 μg of protein was fractionated and used as a sample for two-dimensional electrophoresis.

After adding buffer solution to the sample, protein lysis solution (containing urea and surfactant) was added to make the total volume of 0.34 mL of the sample solution (200 μg of protein). The sample solution was placed in a swelling tray, and a precast gel for first dimension electrophoresis was placed on top of the sample solution. A dry strip cover solution was overlaid and allowed to stand overnight. The swollen precast gels were set on the electrophoresis apparatus and subjected to swimming (500 V for 1 min, 3500 V for 7.5 h, 20°C).

Acrylamide gels with a concentration gradient of 10 to 20% were used. The gels were allowed to stand for 24 hours after preparation to allow the acrylamide to polymerize completely. The equilibrated first dimension electrophoresis precast gel was placed on the acrylamide gel and fixed with 1% agarose solution containing 0.125% bromophenol blue. A molecular weight marker was applied to the left end of the gel to use as a standard for molecular weight. The gel was run at 80 V for 17 hours until the bromophenol blue band was visible at the lower edge of the gel.

After electrophoresis, gels were stained with fluorescent stain for total protein detection (SYPRO Ruby protein gel stain, S21900, Thermo Fisher Scientific Inc.), and images were saved by a fluorescence scanner. Images were captured by the scanner at 488 nm of excitation wavelength, 640 nm Bandpass of fluorescence filter, 100 micrometer of resolution. After the fluorescence-stained images were captured, silver staining was performed using silver nitrate (195-09382, Fujifilm Wako Pure Chemicals Corporation). After development, the silver-stained images were captured using a flatbed scanner.

The results of two-dimensional electrophoresis are shown in Figure 1. In Example 1, three spots (1 to 3) were detected around the isoelectric point pI6 to 9 and the molecular weight of 30,000 to 40,000. In contrast, in Comparative example 1, the three spots detected in Example 1 were not detected. Further, in Comparative Examples 2 and 3, the three spots detected in Example 1 were not detected. It was found that Example 1 contains specific proteins that is not found in Comparative Examples 1 to 3.

(Spot identification)

The spots visualized by silver staining were cut out, and 100 μL of potassium ferricyanide (15 mM) and 100 μL of sodium thiosulfate (50 mM) were added to a 1 mm square gel piece and shaken for 10 minutes. The solution was discarded, Milli-Q water was added and shaken, and the gel was washed until the color was removed from the gel pieces. Acetonitrile was added to the gel pieces and the gels were dehydrated. The gel pieces were swollen by adding 10 μL of an enzyme solution of 100 mM ammonium bicarbonate and 0.01 μg/μL trypsin, and allowed to stand at 37°C for 16 hours. Peptides were extracted by adding 50 μL of 0.1% TFA and 50% acetonitrile and shaking for 20 minutes. Extraction was performed twice. The peptide extract was concentrated by vacuum centrifugation until the peptide extract was reduced to approximately 10 μL. The samples were adsorbed on ZipTip C18 (millipore, ZTC18S960) and the peptides were eluted with 2.5 μL of 60% acetonitrile and 0.1% TFA solution. One μL of the sample solution was mixed with 1 μL of the CHCA matrix solution, dropped onto the target plate, dried, and then measured by a mass spectrometer (ultrafleXtreme). The obtained mass values were used to identify the proteins in the database (NCBI RefSeq). Based on the obtained mass values, the spots were identified from proteins in the database (NCBI RefSeq).

Analyzer: ultrafleXtreme (Bruker Daltonics)

Target plate: MTP Anchorchip 600/384 (209513, Bruker Daltonics)
Polarity: positive mode
Detection mode: reflector mode (300-6,000 m/z)
CHCA matrix solution: 0.3 g/L CHCA, 33% acetone, 66% ethanol
MS/MS Ion Search conditions
Identification software: Mascot (Matrix Science)
Database: NCBI RefSeq
Search species: Bos taurus
Enzyme: Trypsin
Fixation Modification: Carbamidomethylation

The results of the identification of the three spots in Example 1 using the mass spectrometer are shown in Table 1. The spot numbers in Table 1 and Table 2 below correspond to the three spot numbers in Figure 1. The three spots were identified as annexin A2

[Table 1]

| Spot number | Protein | Molecular weight | Isoelectric point |
|---|---|---|---|
| ① | Annexin A2 | 38873 | 7.15 |
| ② | Annexin A2 | 38873 | 7.58 |
| ③ | Annexin A2 | 38873 | 8.07 |

(Calculation of spot signal concentration: numerical analysis of electrophoresis)

Tiff image files of the obtained fluorescence-stained images were imported into ImageMaster Platinum (GE) and analyzed numerically. In the numerical analysis, firstly landmarks were manually assigned to the common spots in each gel. After up to 300 spots per gel were landmarked, the gels were matched. By this process, the spots developed on the gels of both samples were given common spot IDs. Then, spots of the gels were detected and the spot signal concentrations (%volume) were calculated.

The spot signal concentration was calculated from the ratio of the spot signal with respect to the sum of the signals of all the spots in the gel. The %Volume value is expressed as a percentage, and the minimum value is 0.001%.

(Calculation of spot weight)

The protein weight per spot was calculated from the ratio of the weight of 200 $\mu$g of the two-dimensional electrophoresed protein and each spot signal (spot signal concentration) with respect to the sum of spot signals. As a result of calculating the protein weight per spot when 200 $\mu$g of protein was applied to the two-dimensional electrophoresis, in Example 1, the protein weight was calculated to be 0.7 $\mu$g for spot 1, 2.0 $\mu$g for spot 2, and 0.8 $\mu$g for spot 3, for a total of 3.5 $\mu$g for the three spots.

(Calculation of protein weight of three spots in decellularized tissue composition)

The protein weights of the three spots per 20 mg of decellularized tissue composition were calculated from the relationship between the weight of the purified and recovered proteins and the weight of the two-dimensional electrophoresed proteins. The results are shown in Table 2. In Example 1, protein weights were calculated from three spots. Example 1 contained 0.43 % by weight of protein A. In contrast, the protein weight corresponding to the three spots was not calculated in Comparative Example 1. In Comparative Examples 2 and 3, the protein weights corresponding to the three spots were not calculated.

[Table 2]

| Spot number | Example 1 [$\mu$g] | Comparative Example 1 [$\mu$g] | Comparative Example 2 [$\mu$g] | Comparative Example 3 [$\mu$g] |
|---|---|---|---|---|
| ① | 17.7 | 0.00 | 0.00 | 0.00 |
| ② | 47.9 | 0.00 | 0.00 | 0.00 |
| ③ | 20.6 | 0.00 | 0.00 | 0.00 |

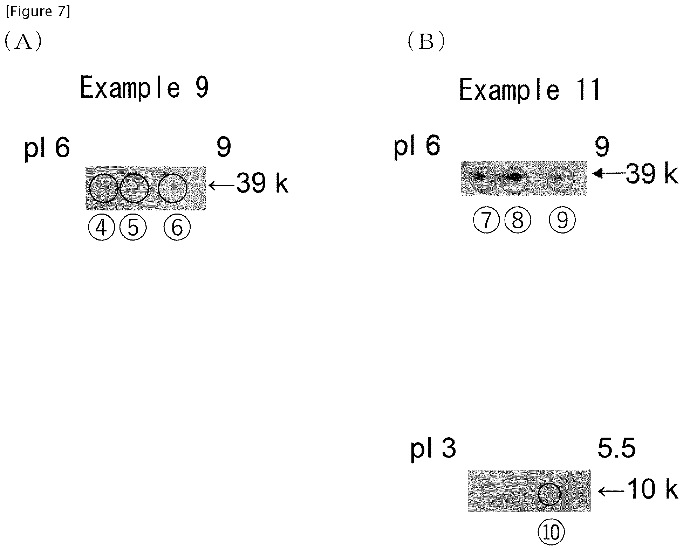

Proteins in the decellularized tissue compositions of Examples 9 and 11 to 13 were similarly analyzed. The result of two-dimensional electrophoresis of Example 9 is shown in Figure 7A. As in Example 1, three spots (4 to 6) were detected around the isoelectric points pI6 to 9 and molecular weights of 30,000 to 40,000. The result of two-dimensional electrophoresis of Example 11 is shown in Figure 7B. Three spots (7 to 9) were detected around the isoelectric point pI6 to 9 and the molecular weight of 30,000 to 40,000, and a spot (10) was detected around the isoelectric point pI4 to 5 and the molecular weight of 8,000 to 12,000. Spot (10) was detected only in Examples 11 to 13.

The spots in Examples 9 and 11 were identified as described above. The result of Example 9 is shown in Table 3. The spot numbers in Table 3 and Table 5 below correspond to the spot numbers in Figure 7A. Spots 4 to 6 in the Example 9 are identified as annexin A2, as in the Example 1 (see spot numbers 1 to 3 in Table 1).

[Table 3]

| Spot number | Protein | Molecular weight | Isoelectric point |
|---|---|---|---|
| ④ | Annexin A2 | 38873 | 7.15 |
| ⑤ | Annexin A2 | 38873 | 7.58 |
| ⑥ | Annexin A2 | 38873 | 8.07 |

The result of Example 11 is shown in Table 4. The spot numbers in Table 4 and Table 6 below correspond to the spot numbers in Figure 7B. Spots 7 to 9 in Example 11 were identified as annexin A2 as in Example 1, and spot 10 was identified as a peptide derived from fibromodulin.

Table 4

| Spot number | Protein | Molecular weight | Isoelectric point |
|---|---|---|---|
| ⑦ | Annexin A2 | 38873 | 7.15 |
| ⑧ | Annexin A2 | 38873 | 7.58 |
| ⑨ | Annexin A2 | 38873 | 8.07 |
| ⑩ | Fragment of fibromodulin | 10000 | 4.65 |

For Examples 9 and 11, the spot signal concentrations and spot weights were calculated as described above.
The protein weight per spot was calculated for each spot when 200 $\mu$g of protein was applied to the two-dimensional electrophoresis. The protein weight in Example 9 was calculated to be 0.14$\mu$g for spot 4, 0.27 $\mu$g for spot 5, and 0.03 $\mu$g for spot 6, for a total of 0.44 $\mu$g for the three spots. The protein weight in Example 11 was calculated to be 0.7$\mu$g for spot 7, 1.6 $\mu$g for spot 8, and 0.4 $\mu$g for spot 9, for a total of 2.7 $\mu$g for the three spots, and 0.24 $\mu$g of protein weight was calculated from spot 10.
For examples 9 and 11, the protein weights of the spots per 20 mg of the decellularized tissue compositions were calculated as described above. The results are shown in Tables 5 and 6. In Example 9, the protein A was contained at 0.05 % by weight. In Example 11, the protein A was contained at 0.33 % by weight, and the protein B was contained at 0.03 % by weight.

[Table 5]

| Spot number | Example 9 [$\mu$g] |
|---|---|
| ④ | 3.4 |
| ⑤ | 6.6 |
| ⑥ | 0.8 |

[Table 6]

| Spot number | Example 11 [$\mu$g] |
|---|---|
| ⑦ | 17.1 |
| ⑧ | 39.3 |
| ⑨ | 9.8 |
| ⑩ | 5.89 |

« Calculation of decellularized DNA ratio »

The masses of the decellularized tissue compositions of Examples 1, 9, and 11 and the decellularized materials of Comparative Examples 1 to 3 were measured, and the samples were immersed in proteolytic enzyme solution to dissolve them. Then, they were treated with phenol/chloroform to remove proteins, and DNAs were recovered. The recovered DNAs were fluorescently stained by Picogreen (Life Technologies) and the DNAs were quantified by measuring the fluorescence intensity. The DNA contents of the samples were calculated from the mass of the samples and the amounts of DNAs. For the quantification of DNA, a calibration curve prepared by using the standard DNA supplied with Picogreen was used. Using samples prepared separately, the dry weight loss ratio was calculated from the mass of the sample (70% moisture content) and the mass of the dried sample (stored in a thermostatic chamber at 60°C for 12 hours). Then, the DNA content per dried mass of the sample was calculated from the DNA content of the sample and the dry weight loss ratio. The results are shown in Table 7.

$$(\text{Decellularized DNA ratio}) = (\text{DNA content of dried specimen of sample})/(\text{mass of sample}) \times 100$$

[Table 7]

|  | DNA content (A) | Sample mass (mg) (B) | Decellularized DNA ratio (A/B x 100; % by weight) |
|---|---|---|---|
| Example 1 | 6012 | 54.4 | 0.0110 |
| Example 9 | 7080 | 50.0 | 0.0142 |
| Example 11 | 7503 | 68.4 | 0.0111 |
| Comparative Example 1 | 27920 | 52.0 | 0.0536 |
| Comparative Example 2 | 18004 | 64.3 | 0.0280 |
| Comparative Example 3 | 9632 | 57.3 | 0.0168 |

It was found that Examples 1, 9, and 11, which were treated with high hydrostatic pressure, showed better decellularization than Comparative Examples.

INDUSTRIAL APPLICABILITY

The decellularized tissue compositions of the present invention can be used as transplantable tissues exhibiting good tissue regeneration.

**Claims**

1. A decellularized tissue composition comprising a decellularized tissue and a protein A with (a) molecular weight of 30,000 to 70,000 and (b) isoelectric point of pI 6.00 to 9.00.

2. The decellularized tissue composition according to claim 1, further comprising a protein B with (a) molecular weight of 3,000 to 15,000 and (b) isoelectric point of pI 3.00 to 5.50.

3. The decellularized tissue composition according to claim 1 or 2, wherein an amount of the protein A is 0.01 to 5.0% by weight with respect to the decellularized tissue composition.

4. The decellularized tissue composition according to any one of claims 1 to 3, wherein an amount of the protein B is 0.001 to 3.0% by weight with respect to the decellularized tissue composition.

5. The decellularized tissue composition according to any one of claims 1 to 4, wherein a mass ratio of protein A and protein B in the composition (A:B) is 5:1 to 150:1.

6. The decellularized tissue composition according to any one of claims 1 to 5, wherein a DNA amount in dry mass is 0.0300 % by weight or less with respect to the decellularized tissue composition.

7. The decellularized tissue composition according to any one of claims 1 to 6, wherein the protein A is annexin.

**8.** The decellularized tissue composition of any one of claims 1 to 7, wherein the protein B is a peptide derived from fibromodulin.

[Figure 1]

(A)

(B)

[Figure 2]

(A)

(B)

(C)

(D)

(E)

[Figure 3]

[Figure 4]

[Figure 5]

Rapture strength [Mpa]

[Figure 6]

Suture strength

[Figure 7]

（A）

## Example 9

pI 6                    9

④⑤  ⑥

←39 k

（B）

## Example 11

pI 6                    9

⑦⑧  ⑨

←39 k

pI 3              5.5

⑩

←10 k

## INTERNATIONAL SEARCH REPORT

International application No.

**PCT/JP2021/041694**

### A. CLASSIFICATION OF SUBJECT MATTER

***C12N 5/071***(2010.01)i
FI: C12N5/071 ZNA

According to International Patent Classification (IPC) or to both national classification and IPC

### B. FIELDS SEARCHED

Minimum documentation searched (classification system followed by classification symbols)

C12N5/071

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | JP 2015-160039 A (UNIV. TOKYO MEDICAL & DENTAL) 07 September 2015 (2015-09-07) | 1-8 |
| A | JP 2018-102491 A (ADEKA CORP.) 05 July 2018 (2018-07-05) | 1-8 |
| A | WO 2016/136633 A1 (ADEKA CORP.) 01 September 2016 (2016-09-01) | 1-8 |
| A | US 2020/0155613 A1 (AMINO TECHNOLOGY LLC) 21 May 2020 (2020-05-21) | 1-8 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **19 January 2022** | **01 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2021/041694**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.

**PCT/JP2021/041694**

| Patent document cited in search report | | | Publication date (day/month/year) | Patent family member(s) | | | Publication date (day/month/year) |
|---|---|---|---|---|---|---|---|
| JP | 2015-160039 | A | 07 September 2015 | (Family: none) | | | |
| JP | 2018-102491 | A | 05 July 2018 | (Family: none) | | | |
| WO | 2016/136633 | A1 | 01 September 2016 | US | 2018/0028722 | A1 | |
| | | | | EP | 3263140 | A1 | |
| US | 2020/0155613 | A1 | 21 May 2020 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005514971 A
- JP 2006507851 A
- JP 2005531355 A